(19) **Europäisches Patentamt**

**European Patent Office**

**Office européen des brevets**

(11) Veröffentlichungsnummer: **0 192 035**
**B1**

(12) **EUROPÄISCHE PATENTSCHRIFT**

(45) Veröffentlichungstag der Patentschrift: **25.04.90**

(21) Anmeldenummer: **86100363.0**

(22) Anmeldetag: **13.01.86**

(51) Int. Cl.⁵: **C 11 B 3/02, C 11 C 3/00**

(54) **Verfahren zur Vorveresterung freier Fettsäuren in Rohfetten und/oder -ölen.**

(30) Priorität: **21.01.85 DE 3501761**

(43) Veröffentlichungstag der Anmeldung:
**27.08.86 Patentblatt 86/35**

(45) Bekanntmachung des Hinweises auf die
Patenterteilung:
**25.04.90 Patentblatt 90/17**

(84) Benannte Vertragsstaaten:
**AT BE DE FR IT NL**

(56) Entgegenhaltungen:
**GB-A- 782 480**
**GB-A-2 145 079**

**R.R. ALLEN et al.: "Bailey's industrial oil and fat products", Band 2, Ausgabe 4, 1982, Seiten 124-127, ausgegeben von D. SWERN, publiziert von J. WILEY & SONS, New York, US**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Jeromin, Lutz, Dr.**
**Am Bandsbusch 88**
**D-4010 Hilden (DE)**
Erfinder: **Peukert, Eberhardt**
**Dürerweg 15**
**D-4010 Hilden (DE)**
Erfinder: **Wollmann, Gerhard, Dr.**
**Kunibertstrasse 25**
**D-4010 Hilden (DE)**

(56) Entgegenhaltungen:
**REVUE FRANCAISE DES CORPS GRAS, Band 32, Nr. 11/12, November-Dezember 1985, Seiten 429-432, Paris, FR; P. MARCHAL et al.: "Neutralisation des huiles végétales brutes par le méthanol, catalysée par des résines échangeuses d'ions"**

**Beschreibung**

Die Erfindung betrifft ein Verfahren zur Vorbehandlung von Triglyceriden, insbesondere Fetten und/oder Ölen nativen Ursprungs zur vereinfachten Beseitigung störender Anteile freier Fettsäuren in solchen Ausgangsmaterialien. Diese Vorbehandlung ist insbesondere als Vorstufe für eine nachfolgende Aufbereitung der nativen Fettstoffe gedacht, bei der durch an sich bekannte Umesterung die Triglyceride mit niederen monofunktionellen Alkoholen, insbesondere mit Methanol zu Glycerin und den entsprechenden Fettsäurealkylestern umgewandelt werden.

Fettsäuremethylester besitzen große technische Bedeutung als Ausgangsmaterial für die Herstellung von Fettalkoholen und anderen fettchemischen Produkten wie Estersulfonaten, Fettsäurealkanolamiden und Seifen. Die industrielle Herstellung der Fettsäuremethylester erfolgt überwiegend durch katalytische Umesterung (Alkoholyse) von Fettsäuretriglyceridgemischen, wie sie in den Fetten und Ölen pflanzlichen und tierischen Ursprungs vorliegen.

Native Fette und Öle enthalten fast immer beträchtliche Mengen freie Fettsäuren, wobei der Gehalt an freien Fettsäuren — je nach Ursprung des Materials und seiner Vorgeschichte — in einem weiten Bereich schwanken kann und fast immer oberhalb von 3 Gewichtsprozent liegt.

Für die Umesterung von natürlich vorkommenden Fettsäuretriglyceriden mit Alkoholen stehen verschiedene Verfahren zur Verfügung. Die Wahl der Verfahrensbedingungen ist in beträchtlichem Ausmaß von der Menge der in den Triglyceriden vorhandenen freien Fettsäuren abhängig.

In Gegenwart von alkalischen Katalysatoren können Fette und Öle bei 25 bis 100°C mit einem 0,5 bis 1.0-molaren Überschuß an Alkohol unter Normaldruck zu den entsprechenden Fettsäureestergemischen umgeestert werden. Ein entsprechendes Verfahren ist als erste Stufe einer Seifenherstellung in der US—PS 2 360 844 beschrieben. Diese alkalikatalysierte drucklose Umesterung kann problemlos durchgeführt werden, solange man von Fetten und Ölen ausgeht, die weitgehend vasserfrei sind und deren Gehalt an freien Fettsäuren unter 0,5 Gewichtsprozent (entsprechend einer Säurezahl von etwa 1) liegt.

Nach einem anderen Verfahren können auch Fette und Öle mit einem höheren Gehalt an freien Fettsäuren in Gegenwart von Alkalioder Zink-Katalysatoren bei 240°C unter einem Druck von etwa 100 bar mit einem 7- bis 8-molaren Überschuß an Methanol zu den entsprechenden Fettsäuremethylestern umgeestert werden (Ullmann, Enzyklopädie der technischen Chemie, 4. Auflage, Band 11 (1976), Seite 432).

Gegenüber der Umesterung unter erhöhtem Druck zeichnet sich die drucklose Umesterung durch einen erheblich geringeren Methanolbedarf und — wegen der niedrigeren Reaktionstemperaturen — durch einen geringen Energieaufwand aus Darüber hinaus kommt die Umesterung unter Normaldruck ohne kostspielige Druckreaktoren aus. Aufgrund der Tatsache, daß in technischen Fetten und Ölen fast immer größere Mengen an Wasser und Fettsäuren vorliegen. setzt die drucklose Umesterung eine Trocknung sowie eine Verringerung der Säurezahl — z.B. durch vorhergehende Umwandlung der freien Fettsäuren in die entsprechenden Alkyl- oder Glycerinester im Zuge einer Vorveresterungsreaktion — voraus.

Die Vorveresterung der säurehaltigen Fette und Öle kann in Gegenwart von alkalischen Katalysatoren bei 240°C und 20 bar durchgeführt werden (Ullmann. Enzyklopädie der technischen Chemie. 4. Auflage, Band 11 (1976). Seite 432). Diese Art der Vorveresterung mit Methanol setzt wiederum die Verwendung von kostspieligen Druckreaktoren voraus.

Bekannt ist auch die Veresterung der freien Fettsäuren im Öl mit zugesetzten monofunktionellen niederen Alkoholen, insbesondere Methanol, in homogener phase mittels saurer Katalyse z. B. unter Mitverwendung von p-Toluolsulfonsäure als Katalysator. Dieses Verfahren erfordert allerdings eine relativ schwierige Katalysatorabtrennung mit gleichzeitiger Wasserentfernung durch eine Wäsche des vorveresterten Öls mit Methanol. Urn das Waschmethanol mit dem Wasser und Katalysator abtrennen zu können, muß schon zur Vorveresterung ein mit der Ölphase im wesentlichen nicht mischbares flüssiges Schleppmittel dem Öl zugemischt werden, wobei bevorzugt freies Glycerin als Schleppmittel eingesetzt wird. Ein entsprechendes Verfahren ist in der DE-OS 33 19 590 beschrieben. Bei diesem Verfahren treten allerdings Verluste an Estern der freien Säuren auf. Die sorgfältige Entfernung des Katalysators — üblicherweise p-Toluolsulfonsäure — ist unbedingt erforderlich, da Katalysatorreste im Methylester bei dessen nachfolgender Umsetzung zum Fettalkohol die Hydrierkatalysatoren inhibieren können.

Die GB-PS 782 480 beschreibt ein Verfahren zur Herstellung von Diol- oder Triolmonocarbonsäureestern, bei dem man die Diole oder Triole in Gegenwart von sauren Ionenaustauscherharzen miteinander umsetzt. Die Ionenaustauscherharze können als polare Gruppen Sulfonsäure-, Carbonsäure- oder Phosphonsäuregruppen sowie phenolische Gruppen enthalten. In dem Verfahren werden die in Substanz vorliegenden Ausgangsmaterialien Polyol und Carbonsäure miteinander umgesetzt. Bei der Durchführung der Umsetzung wird das gebildete Reaktionswasser durch azeotrope Destillation mit Toluol laufend aus dem Reaktionsgemisch entfernt. Die Entsäuerung von Fetten und Ölen wird in der Patentschrift nicht angesprochen.

Bailey's Industrial Oil and Fat Products. Band 2. Seiten 124—126 erwähnt die Verwendung von Ionenaustauscherharzen als Veresterungskatalysatoren im Zusammenhang mit einer Mehrzweckapparatur, die für die verschiedenen mit Fettsäuren durchzuführenden Veresterungs- und Umsetzungsverfahren geeignet ist. Aus dem Aufbau der Apparatur (Figur 2.8 auf Seite 125) mit azeotroper storage, aufgesetzter Destillationskolonne und Wasserabscheider kann entnommen werden, daß hier im Falle einer Veresterungsreaktion die Entfernung des Reaktionswassers durch azeotrope Destillation vorgesehen ist. Auch hier ist die Entsäuerung von Fetten und Ölen nicht erwähnt.

Die Erfindung geht von der Aufgabe aus ein Verfahren zu entwickeln. das alle Vorteile eines sauer katalysierten Vorveresterungsverfahrens — insbesondere das Arbeiten bei vergleichsweise milden Temperaturen und im Bereich von Normaldruck — beinhaltet und gleichwohl frei ist von den bisherigen Schwierigkeiten der zuverlässigen Beseitigung unerwünschter Restanteile des sauren Katalysators aus dem behandelten Gut. Zur Lösung dieser Aufgabenstellung schlägt die Erfindung vor, die Stufe der Vorveresterung in heterogener Phase derart durchzuführen, daß mit sauren Feststoffkatalysatoren gearbeitet, wird, die sich in dem zu behandelnden Produktstrom nicht losen und infolgedessen durch einfache Phasentrennung zuverlässig vom Reaktionsgemisch abgetrennt werden können.

Gegenstand der Erfindung ist dementsprechend ein Verfahren zur Senkung des Gehalts an freien Säuren in Fetten und/oder Ölen durch deren Behandlung mit einem $C_{1-4}$ Monoalkohol in Gegenwart saurer Veresterungskatalysatoren, das dadurch gekennzeichnet ist, daß man als Katalysator feste Kationenaustauscherharze in saurer Form einsetzt und die Entfernung des Reaktionswassers nach der Abtrennung des Reaktionsgemisches von dem Kationenaustauscherharz vornimmt.

Bevorzugte Katalysatoren der Erfindung sind stark saure Kationenaustauscherharze, die freie Sulfonsäuregruppen an eine Polymermatrix gebunden enthalten. Entsprechende Ionenaustauscherharze sind in vielgestaltiger Form unter zahlreichen Handelsnamen bekannt. Erfindungsgemäß geeignet sind insbesondere entsprechende Harztypen mit makroporöser Struktur, die den intensiven Kontakt zwischen Ölphase und heterogener Feststoffphase erleichtern. Einschlägige Literaturhinweise zu diesen Ionenaustauscherharzen mit Resten starker Säuren als Ionenaustauschergruppierungen — insbesondere Sulfonsäurresten — finden sich beispielsweise in Ullmanns Encyclopädie der technischen Chemie, 3. Auflage, 8. Band. München-Berlin 1957, Seiten 806 bis 817. dort insbesondere Tabelle 3, Seite 816.

Die erfindungsgemäße Durchführung der Vorveresterung freier Fettsäuren in den Rohfetten und/oder -ölen mittels heterogener Feststoffkatalyse an Kationenaustauscherharzen verbindet die zuvor geschilderten Vorteile der sauren Katalyse mit der absolut zuverlässigen Abtrennbarkeit des Katalysators aus dem Reaktionsgemisch. Hierdurch werden bedeutende Verfahrensvereinfachungen und Einsparungen ermöglicht.

Als Ausgangsmaterialien für das erfindungsgemäße Verfahren eignen sich native Fette und/oder Öle, die als solche, d. h. im ungereinigten Zustand, dem Verfahren unterworfen werden können. Überraschenderweise zeigt sich auch im kontinuierlichen Verfahren nach lang anhaltenden Verfahrensperioden kein oder kein wesentlicher Aktivitätsverlust an den heterogenen Feststoffkatalysatoren. Gegebenenfalls auftretende Aktivitätsverluste können durch Spülung der Katalysatorphase insbesondere mit dem zur Vorveresterung eingesetzten monofunktionellen Alkohol und erforderlichenfalls durch Reaktivierung der sauren Ionenaustauschergruppen im Harz wieder beseitigt werden.

Als Ausgangsmaterial für das erfindungsgemäße Verfahren kommen praktisch alle Fette und Öle pflanzlichen oder tierischen Ursprungs in Betracht, soweit ihr Gehalt an freien Fettsäuren nicht von Natur aus so gering ist, daß sie ohne Nachteil der alkalikatalysierten drucklosen Umesterung direkt zugeführt werden können. Zu den möglichen Ausgangsmaterialien zählen insbesondere Kokosöl, Palmkernöl, Olivenöl, Rapsöl, Baumwollsaatöl, Schmalzöl, Fischöl und Rindertalg. Die Säurezahl der natürlichen Fette und Öle und damit ihr Gehalt an freien Fettsäuren kann in weiten Bereichen schwanken. So liegt beispielsweise die Säurezahl des handelsüblichen rohen Kokosöls in der Regel nicht über 20. Bei anderen Pflanzenölen liegt die Säurezahl guter Qualitäten unterhalb von 10; bei minderen Qualitäten können Werte von 20 bis 25 beobachtet werden. Technische Talge. die nach ihrer Säurezahl bewertet und gehandelt werden, besitzen — einem Gehalt an freien Fettsäuren von 1 bis 20 Gewichtsprozent entsprechend — Säurezahlen von 1 bis 40, wobei gelegentlich auch noch höhere werte auftreten können. In extremen Fällen kann die Säurezahl des Ausgangsmaterials für das erfindungsgemäße Verfahren werte von 60 und darüber erreichen.

Im erfindungsgemäßen Verfahren wird der in den Triglyceridgemischen des Ausgangsmaterials vorhandene Anteil an freien Fettsäuren in Gegenwart der sauren Kationenaustauscherharze mit im Überschuß vorhandenem Monoalkohol ($C_{1-4}$-Alkohole, vorzugsweise Methanol) verestert. Dazu werden vergleichsweise milde Reaktionsbedingungen gewählt, so daß eine Umesterung der Triglyceride mit dem Monoalkohol nicht oder nur in begrenztem Ausmaß stattfindet. Das Verhältnis zwischen Triglycerid und dem Monoalkohol wird dabei zweckmäßigerweise so gewählt, daß einerseits ein deutlicher Alkoholüberschuß über die zu veresternde freie Säure vorhanden ist, und daß andererseits am Ende der Reaktion eine ungestörte Aufarbeitung gewährleistet ist. Zu diesem Zweck werden normalerweise auf 100 Volumenteile Ausgangsmaterial 10 bis 50 Volumenteile $C_{1-4}$-Monoalkohol eingesetzt.

Die Vorveresterung wird in der Regel bei Normaldruck durchgeführt. Das Arbeiten bei geringem Überdruck kann in verschiedenen Fällen von Vorteil sein. Es kommen dann aber nur Drucke bis zu 5 bar in Betracht, zu deren Realisierung keine speziellen Druckreaktoren erforderlich sind.

Als Reaktionstemperatur für die Durchführung der Vorveresterung ist bei Drucken im Bereich des Normaldrucks insbesondere der Temperaturbereich in der Nähe des Siedepunktes des eingesetzten Monoalkohols geeignet, wobei Reaktionstemperaturen zwischen dem Siedepunkt und 10°C darunterliegenden Temperaturen geeignet sein können. Beim Arbeiten mit Methanol werden dementsprechend bevorzugt Temperaturen im Bereich von 55 bis 65°C eingehalten.

Um das Reaktionswasser zu entfernen, das bei der Veresterung entsteht und bei der nachfolgenden Umesterung des Öls zu Methylester stören würde, wird das vom Kationenaustauscherharz abgetrennte Reaktionsgemisch einer Trocknung unterworfen, bevor der im Kreislauf geführte Teilstrom rückgeführt oder das Reaktionsgemisch weiterverarbeitet wird. Der Ölstrom kann beispielsweise über einen Fallfilmverdampfer geleitet werden. Das Reaktionswasser mit dem Restmethanol geht hier in die Dampfphase über. wird kondensiert und damit dem Prozeß entzogen Eine Rektifikation des Alkohol/wasser-Gemisches ist möglich, wodurch die Rückführung des Alkoholanteiles in den Verfahrenskreislauf gelingt. Die Trocknung des Produktstromes ist jedoch auch auf andere an sich bekannte weise möglich, so kann beispielsweise der vom Feststoffkatalysator abgezogene Produktstrom über ein Trocknungsmittel — beispielsweise ein Molekularsieb — geleitet werden, wodurch das Reaktionswasser aus dem Kohlenwasserstoffgemisch herausgenommen wird.

Es hat sich als vorteilhaft erwiesen. zur Verbesserung der Strömungs- und auch der Stoffübergangsbedingungen am heterogenen Feststoffkatalysator dem Reaktionsgemisch ein mit letzterem mischbares Verdünnungsmittel zuzugeben. Als Verdünnungsmittel im Rahmen der Erfindung eignet sich ein rückgeführter Teilstrom des Reaktionsgemischs und/oder ein Teilstrom eines Fettsäurealkylesters, der beispielsweise aus der nachfolgenden Umesterungsstufe abgezweigt werden kann. Das Verdünnungsmittel wird dem zu behandelnden Stoffgemisch vor dessen Kontakt mit dem Feststoffkatalysator zugesetzt. Es kann dabei beispielsweise in Mengen von 30 bis 500 Volumenanteilen Verdünnungsmittel auf 100 Volumenteile Ausgangsmaterial/Alkohol-Gemisch zugegeben werden, wobei insbesondere das Arbeiten mit etwa 50 bis 200 Volumenteilen Verdünnungsmittel auf 100 Volumenteile Ausgangsmaterial/Alkohol-Gemisch bevorzugt ist.

Die heterogenen festen Kationenaustauscherharze können im erfindungsgemäßen Verfahren insbesondere als Festbett eingesetzt werden, durch das der zu behandelnde Strom des flüssigen Gutes geleitet wird. Die heterogene Katalyse kann aber auch in einer bewegten Schüttung zum Beispiel im Fließbett und/oder im wirbelbett erfolgen, vorausgesetzt. daß durch einen nachfolgenden Trennschritt die zuverlässige Abtrennung des Feststoffkatalysators von der Flüssigphase gewährleistet ist. Besonders geeignete Katalysatoren für die erfindungsgemäße heterogene Katalyse sind stark saure, makroporöse, in wasserfreien und unpolaren Medien einsetzbare Kationenaustauscher, wie sie beispielsweise unter dem warenzeichen Lewatit(R), und zwar insbesondere der Typen SPC 118 BG, SPC 118, SPC 108 BG und SPC 108 von der Firma Bayer AG vertrieben werden.

Mit diesen Feststoffkatalysatoren wird im erfindungsgemäßen Verfahren das Rohöl beispielsweise bei einer Temperatur von 60 bis 65°C zusammen mit Methanol durch eine Säule mit Festbett gepumpt, das aus Katalysatorharz besteht. Die Säurezahl der Fette und/oder Öle wird in der Regel auf werte von 1 oder darunter abgesenkt. So kann beispielsweise beim Arbeiten mit Kokosöl die Säurezahl von 10 im Rohöl auf werte unter 1 abgesenkt werden. Im bevorzugten Verfahren beträgt das Katalysatorvolumen bezogen auf den Öldurchsatz 1 bis 10 1 Harz pro Liter Rohöl pro Stunde, wobei insbesondere mit Verhältnissen von 1.5 bis 7.5 1 Harz pro Liter Rohöl pro Stunde gearbeitet werden kann. In der Praxis kann die Vorveresterung und teilweise Umesterung beispielsweise derart erfolgen, daß der zu behandelnde Flüssigkeitstrom durch ein oder zwei hintereinander geschaltete lonenaustauschersäulen geleitet wird, die auf Reaktionstemperatur beheizt sind. Die Flüssigphase durchströmt die lonenaustauschersäulen bevorzugt von unten nach oben. Gegebenenfalls entstehende gasförmige Anteile — Luftblasen oder verdampfter Alkohol — durchwandern die Katalysatorschüttung, um am Kopf der Kolonne kondensiert oder abgeblasen zu werden. Siebe am Kopf der Kolonne verhindern den Austrag des lonenaustauscherharzes. Aus dem vorveresterten Öl trennen sich die Schlamm- und Schleimstoffe, die im Rohöl enthalten sind. Der produktstrom wird wie zuvor beschrieben von seinem Wassergehalt und gegebenenfalls Restgehalten an freiem Alkohol befreit. Ein Teilstrom des gereinigten, getrockneten, vorveresterten Öles kann als Verdünnungsmittel indie Vorveresterung zurückgeleitet werden.

Beispiele
Beispiel 1

Die Vorveresterung erfolgte kontinuierlich in zwei hintereinander geschalteten lonenaustauschersäulen von 100 mm Durchmesser, die mit je 3,5 l lonenaustauscherharz Lewatit(R) SPC 118 BG gefüllt waren, Über eine Mantelheizung waren beide Säulen auf eine Temperatur von 64°C temperiert.

Vorgewärmt auf die Reaktionstemperatur von 64°C wurden 0,2 l/h Methanol vermischt mit 1 l/h ungereinigtem Kokosöl mit einer Säurezahl von 10, zusammen mit 0,98 l/h Kreislaufprodukt von unten nach oben durch die Säulen gepumpt. Um das Kreislaufprodukt wasserfrei zurückführen zu können, wurde der aus den Säulen austretende Produktstrom über einen Fallfilmverdampfer geleitet. der als Umlaufverdampfer bei Umgebungsdruck eine Temperatur der Flüssigphase von 120°C erforderte. Das wasserfreie und vom Überschußmethanol befreite Öl hatte eine Säurezahl von 0,55 und einen Wassergehalt von 0,08 Gew.-%.

In einem Abscheider wurden die Schlamm- und Schleimstoffe, die im Rohöl enthalten waren, abgetrennt. Ein Teilstrom von 0,98 l des auf diese Weise kontinuierlich vorveresterten Produktes wurde als Kreislaufstrom zurückgeführt. Die übrigen Anteile wurden der Umesterung zugeführt.

### Beispiel 2

4,4 l Ionenaustauscherharz Lewatit(R) SPC 118 BG wurden in ein beheizbares Rohr von 30 cm Innendurchmesser eingefüllt. 3 l/h ungereinigtes Kokosöl mit einer Säurezahl von 10 wurde mit 0,6 l/h Methanol vermischt und im Durchlauf durch einen wärmetauscher auf eine Temperatur von 64°C erhitzt.

Nach einmaliger Durchströmung des auf eine Temperatur von 62°C temperierten Festbettes hatte das Gemisch eine Säurezahl von 0,56.

### Beispiel 3

In einem Vergleichsversuch wurden die Ionenaustauscher Lewatit(R) SPC 108, SPC 118 und SPC 118 BG untersucht. Durch eine beheizte Säule (64°C) mit jeweils 517 g Harz wurde ein Gemisch aus 1800 ml Kokosöl, 900 ml MeOH und 460 ml Methylester mit einer Umlaufgeschwindigkeit von 25 l/h im Kreis gepumpt.

Das Gemisch enthielt bei Reaktionsbeginn einen Anteil an freien Fettsäuren. Die Säurezahl betrug 3,2.

Nach 2 Stunden Umpumpen wiesen die Gemische nach der Säule Säurezahlen von 0,91, 1,15 und 0.89 auf. Nach 6 Stunden Umpumpen lagen die Säurezahlen bei 0,32, 0,42 und 0,41.

## Patentansprüche

1. Verfahren zur Senkung des Gehalts an freien Säuren in Fetten und/oder Ölen durch deren Behandlung mit einem $C_{1-4}$-Monoalkohol in Gegenwart saurer Veresterungskatalysatoren, dadurch gekennzeichnet, daß man als Katalysator feste Kationenaustauscherharze in saurer Form einsetzt und die Entfernung des Reaktionswassers nach der Abtrennung des Reaktionsgemisches von dem Kationenaustauscherharz vornimmt.

2. Verfahren nach Anspruch 1, dadurch gekennzeichnet, daß man mit stark sauren Kationenaustauscherharzen arbeitet, die freie Sulfonsäuregruppen an einer Polymermatrix enthalten.

3. Verfahren nach Ansprüchen 1 und 2, dadurch gekennzeichnet, daß man native Fette und/oder Öle im nicht vorbehandelten Zustand dem Verfahren unterwirft.

4. Verfahren nach Ansprüchen 1 bis 3, dadurch gekennzeichnet, daß die $C_{1-4}$-Monoalkohole in Mengen von 10 bis 50 Volumenteilen pro 100 Volumenteile Ausgangsmaterial eingesetzt werden.

5. Verfahren nach Anspruch 4, dadurch gekennzeichnet, daß als Monoalkohol Methanol eingesetzt wird.

6. Verfahren nach Ansprüchen 1 bis 5, dadurch gekennzeichnet, daß man ein werdünnungsmittel zusammen mit dem Ausgangsmaterial/Alkohol-Gemisch über das Kationenaustauscherharz leitet, wobei ein im Kreislauf geführter Teilstrom des Reaktionsgemischs und/oder ein Teilstrom eines Fettsäurealkylesters als Verdünnungsmittel eingesetzt werden.

7. Verfahren nach Ansprüchen 1 bis 6, dadurch gekennzeichnet, daß das vom Kationenaustauscherharz abgezogene Reaktionsgemisch einer Trocknung unterworfen wird, bevor der im Kreislauf geführte Teilstrom rückgeführt oder das Reaktionsgemisch weiterwerarbeitet wird.

8. Verfahren nach Ansprüchen 1 bis 7, dadurch gekennzeichnet, daß bei Drucken im Bereich des Normaldrucks und Temperaturen in der Nähe des Siedepunkts des Monoalkohols gearbeitet wird.

9. Verfahren nach Anspruch 8, dadurch gekennzeichnet, daß beim Arbeiten mit Methanol Temperaturen im Bereich von 55 bis 65°C eingehalten werden.

10. Verfahren nach Ansprüchen 1 bis 9, dadurch gekennzeichnet, daß das Verdünnungsmittel in Mengen von 30 bis 500 Volumenteilen auf 100 Volumenteile Ausgangsmaterial/Alkohol-Gemisch zugegeben wird.

11. Verfahren nach Anspruch 10, dadurch gekennzeichnet, daß das Verdünnungsmittel in Mengen von 50 bis 200 Volumenteilen auf 100 Volumenteile Ausgangsmaterial/Alkohol-Gemisch zugegeben wird.

12. Verfahren nach Ansprüchen 1 bis 11, dadurch gekennzeichnet, daß das Verfahren so lange durchgeführt wird, bis die Säurezahl der Fette und/oder Öle auf Werte von 1 oder darunter gesenkt ist.

## Revendications

1. Procédé en vue d'abaisser la teneur en acides libres dans des graisses et/ou des huiles en les traitant avec un mono-alcool en $C_1$—$C_4$ en présence de catalyseurs acides d'estérification, caractérisé en ce que, comme catalyseur, on utilise des résines échangeuses de cations solides sous forme acide et en ce qu'on effectue l'élimination de l'eau réactionnelle après séparation du mélange réactionnel de la résine échangeuse de cations.

2. Procédé selon la revendication 1, caractérisé en ce qu'on travaille avec des résines échangeuses de cations fortement acides contenant des groupes d'acides sulfoniques libres sur une matrice polymère.

3. Procédé selon les revendications 1 et 2, caractérisé en ce qu'on soumet, au procédé, des huiles et/ou des graisses naturelles à l'état non prétraité.

4. Procédé selon les revendications 1 à 3, caractérisé en ce qu'on utilise le mono-alcool en $C_1$—$C_4$ en quantités de 10 à 50 parties en volume par 100 parties en volume de matière de départ.

5. Procédé selon la revendication 4, caractérisé en ce que, comme mono-alcool, on utilise le méthanol.

6. Procédé selon les revendications 1 à 5, caractérisé en ce qu'on fait passer un diluant conjointement avec le mélange de matière de départ/ alcool sur la résine échangeuse de cations en utilisant, comme diluant, un courant partiel mis en cycle du mélange réactionnel et/ou un courant partiel d'un ester alkylique d'acide gras.

7. Procédé selon les revendications 1 à 6, caractérisé en ce qu'on soumet le mélange réactionnel retiré de la résine échangeuse de cations à un séchage avant de recycler le courant partiel mis en cycle ou de soumettre le mélange réactionnel à un traitement complémentaire.

8. Procédé selon les revendications 1 à 7, caractérisé en ce qu'on travaille sous des pressions se situant dans le domaine de la pression normale et des températures voisines du point d'ébullition du mono-alcool.

9. Procédé selon la revendication 8, caractérisé en ce que, lorsqu'on travaille avec du methanol, on maintient des températures se situant dans l'intervalle allant de 55 à 65°C.

10. Procédé selon les revendications 1 à 9, caractérisé en ce qu'on ajoute le diluant en quantités de 30 à 500 parties en volume pour 100 parties en volume du mélange de matière de dé part/alcool.

11. Procédé selon la revendication 10, caractérisé en ce qu'on ajoute le diluant en quantités de 50 à 200 parties en volume pour 100 parties en volume du mélange de matière de départ/alcool.

12. Procédé selon les revendications 1 à 11, caractérisé en ce qu'on effectue le procédé jusqu'à ce que l'indice d'acide des graisses et/ou des huiles soit abaissé à des valeurs de 1 ou moins.

## Claims

1. A process for reducing the content of free fatty acids in fats and/or oils by treatment thereof with a $C_{1-4}$ monoalcohol in the presence of acidic esterification catalysts, characterized in that solid cation exchanger resins in acidic form are used as the catalyst and the water of reaction is removed after separation of the reaction mixture from the cation exchanger resin.

2. A process as claimed in claim 1, characterized in that strongly acidic cation exchanger resins containing free sulfonic acid groups on a polymer matrix are used.

3. A process as claimed in claims 1 and 2, characterized in that native fats and/or oils are subjected to the process in non-pretreated form.

4. A process as claimed in claims 1 to 3, characterized in that the $C_{1-4}$ monoalcohols are used in quantities of 10 to 50 parts by volume per 100 parts by volume starting material.

5. A process as claimed in claim 4, characterized in that methanol is used as the monoalcohol.

6. A process as claimed in claims 1 to 5, characterized in that a diluent is passed over the cation exchanger resin together with the mixture of starting. material and alcohol, a circulated partial stream of the reaction mixture and/or a partial stream of a fatty acid alkyl ester being used as the diluent.

7. A process as claimed in claims 1 to 6, characterized in that the reaction mixture separated from the cation exchanger resin is subjected to drying before the circulated partial stream or the reaction mixture is further processed.

8. A process as claimed in claims 1 to 7, characterized in that it is carried out under pressures around normal pressure and at temperatures around the boiling point of the monoalcohol.

9. A process as claimed in claim 8, characterized in that, where methanol is used, the process is carried out at temperatures in the range from 55 to 65°c.

10. A process as claimed in claims 1 to 9, characterized in that the diluent is added in quantities of 30 to 500 parts by volume to 100 parts by volume of the starting material/alcohol mixture.

11. A process as claimed in claim 10, characterized in that the diluent is added in quantities of 50 to 200 parts by volume to 100 parts by volume of the starting material/ alcohol mixture.

12. A process as claimed in claims 1 to 11, characterized in that it is carried out until the acid value of the fats and/or oils has fallen to values of 1 or lower.